# EUROPEAN PATENT SPECIFICATION

(11) **EP 1 385 499 B1**
(45) Date of publication and mention of the grant of the patent: **09.07.2008**
(21) Application number: 02742878.8
(22) Date of filing: 08.04.2002
(51) Int. Cl.: A61K 31/205, A61P 9/04

(54) **USE OF CARNITINE FOR THE PREPARATION OF AN INJECTION SOLUTION FOR PREVENTING OR ALLEVIATING ASCITES IN POULTRY BY IN-OVO INJECTION**
VERWENDUNG VON CARNITIN ZUR HERSTELLUNG EINER INJEKTIONSLÖSUNG ZUR VORBEUGUNG ODER VERMEIDUNG VON ASZITES VON GEFLÜGEL DURCH IN-OVO-INJEKTION
UTILISATION DU CARNITINE POUR LA PREPARATION D'UNE INJECTION POUR LA PREVENTION OU L'AMELIORATION DE L'ASCITE POUR LA VOLAILLE PAR INJECTION IN OVO

(30) Priority: 09.04.2001 US 282447 P
(43) Date of publication of application: 04.02.2004
(73) Proprietor: Lonza AG, 4052 Basel (CH); THE BOARD OF REGENTS OF OKLAHOMA STATE UNIVERSITY, Stillwater, OK 74078 (US)
(72) Inventor: TEETER, Robert, G., Stillwater, OK 74074 (US); VANHOOSER, Stanley, L., Glencoe, OK 74032 (US); OWEN, Kevin, Q., Canyon, TX 79015 (US)
(74) Representative: Riegler, Norbert Hermann
(86) International application number: PCT/EP2002/003878
(87) International publication number: WO 2002/080900

(56) References cited:
- EP-A- 0 620 981
- WO-A-00/56330
- WO-A-02/12436
- US-A- 5 889 055
- TKAHASHI, ICHIMATSU: "Embryochemische Untersuchungen mittels der Injektionsmethode VI. Über das Verhalten des Kreatins bzw. Kreatinins im Hühnerei bei der Aminosäureinjektion" ZEITSCHRIFT FÜR PHYSIOLOGISCHE CHEMIE, vol. 219, 1933, pages 31-33, XP001098670
- DATABASE WPI Section Ch, Week 199412 Derwent Publications Ltd., London, GB; Class B05, AN 1994-098705 XP002211067 & SU 1 790 382 A (UNIV MARIISK), 23 January 1993 (1993-01-23)

## Description

The present invention is in the general field of poultry farming and relates to improving the hatchability of eggs and/or preventing or alleviating ascites in poultry.

During recent decades the ascites syndrome has evolved into a significant threat to high yielding broilers. This is especially true for segments of the broiler industries located in either cold and/or high altitude regions; pulmonary damages either by disease or dust and ammonia resulting from mass raising of poultry also predispose to the disease. At the onset of ascites, birds initially compensate for increased metabolic demand of oxygen by increasing cardiac output. However, problems arise when the return blood volume exceeds the capacity of their heart to pump the blood. As a result, the heart enlarges, right heart ventricles dilating. Pathologically evolving cardiac traits entail changes in haematocrit, liver congestion, increased vena cava pressure and the accumulation of interstitial fluids in the abdominal cavities due to transsudation from the liver.

Sudden death syndrome has been recognized in recent years also to be caused by similar cardiac deformations. It is endemic in large, industrially raised stocks of poultry and shares a common physiological cause with ascites.

WO 00/56330 and WO 00/56294 of the present applicant teach that supplementing the diet of broiler-type poultry will prevent and alleviate ascites and sudden death syndrome.

As a disadvantage however, carnitine is costly as a feed compound that is fed on a regular, daily scheme. Overdosing is likely to occur since the precise amount of feed ingested by the birds can vary from day to day, rendering optimal dosing even more difficult.

It is an object of the present invention to avoid the disadvantages of the prior art and to devise another method that allows for the improvement of the well-being of poultry by means of admininstration of carnitine while being more efficient with regard to the amount of carnitine needed to achieve such improvement.

This object is achieved by the method or use according to the following description and claims.

Improving the well-being of poultry and/or preventing or alleviating ascites in poultry is provided comprising the step of providing to embryonated eggs laid by a breeder hen an effective amount of carnitine by means of in-ovo injection.

It has been found that a single injection of carnitine into incubating eggs increased body weight at hatching by 0.5 g. Most surprisingly, this increase carried through to other ages; the effect of in-ovo injection of carnitine was present at 21 days and 49 days post-hatch, as well as weight gain in all periods measured. The feed conversion ratio was found to be improved as well.

Furthermore, the mortality post-hatch was numerically reduced by in-ovo injection as compared to control animals hatched from non-injected eggs. The effect was most pronounced at higher ages than immediately post-hatch, which is particularly surprising. The mortality rate was reduced to approximately cumulative 20% for the highest age.

Finally, employing controlled testing conditions of metabolic stress due to lowered oxygen supply, ascites related physiological parameters such as haematocrit and ascites score were found to be improved by in-ovo injection. Given a common cause in cardiomyopathy and the development of abnormal cardiac traits and cardiac lesions, the present invention should be considered to be applicable in preventing or alleviating ascites and/or sudden death syndrome, and the mention of either condition herein should be taken to also refer to the other.

A better understanding of the present invention and its objects and advantages will become apparent to those skilled in this art from the following detailed description, wherein there is described only the preferred embodiment of the invention.

In the context of the present invention, the ascites score is understood as an empirical score from 0 to 3 indicating no to severe myocardial degeneration, based on the histopathological changes. Pathological changes in cardiac traits such as right ventricle weight and ascites heart ratio, defined as the ratio of right ventricle weight to total ventricle weight, are also taken into account.

A score of zero indicated minimal fat deposition and myofiber size variation, whereas a score of three indicated marked myocardial degeneration, highly abnormal cardiac traits and arteritis. A score of two was intermediate to the two extremes.

Apart from the ascites score, hematocrit values are the most direct reflection of cardiac function. Elevated blood hematocrit and elevated ascites heart ratio are symptoms of the cardiac malfunction causing ascites, i.e. the accumulation of serous fluids in the abdominal cavities between the organs.

It is most surprising that a single injection of an effective amount of carnitine in-ovo, pre-hatch, is sufficient to reduce the extend and/or incidence of cardiomyopathy, such cardiomyopathy eventually occuring weeks later post-hatch, in the course of rapid growth of the young animal. Without being bound to theory, a possible explanation for this may be seen in chick body temperature at hatching which has been hypothesized to be a significant parameter of ascites resistance and subsequent growth performance.

Suitable "poultry" according to the present invention is used herein in its normal broad sense, referring to birds of several species, and includes e.g. chickens, turkeys, ducks, pigeons, geese, swans, guineas, pigeons, peafowl, ostriches, pheasants, quail or any other poultry species which has female breeders hens.

Eggs according to the present invention preferably are embryonated eggs, i.e. eggs that are properly incubated to give rise to offspring.

The carnitine provided to the eggs can be any kind of carnitine or carnitine derivative or a salt thereof which is non-toxic and pharmaceutically acceptable. Carnitine, chemically known as 3-hydroxy-4-N-trimethylaminobutyric acid or, alternatively, β-hydroxy-γ-trimethylaminobutyric acid, is a quaternary ammonium compound present in vertebrate muscle whose metabolic friction involves the transfer of fatty acids across mitochondrial membranes. Preferably, the carnitine is L-Carnitine which is the naturally occuring physiologic form.

Suitable carnitine derivatives can be e.g. 3-alkanoyl-derivatives such as acetyl-carnitine or propionyl-carnitine. Suitable salts can be e.g. L-carnitine inner salt, L-carnitine tartrate, L-carnitine fumarate or L-carnitine magnesium citrate. However, carnitine according to the present invention may as well be coated on a suitable inert or soluble carrier such as e.g. a fine-grinded silicate or starch particles.

For in-ovo-injection, the carnitine according to the present invention is, e.g., freshly suspended or dissolved in a suitable solvent such as e.g. a physiological (0.7% w/w) saline solution or sterile water. Expediently, such solvent or solution has been sterilized prior to or is sterilized after carnitine addition. Sterilization can be performed by means well known such as, e.g., autoclaving or filtration through 2p-filters.

The amount of carnitine in-ovo-injected is an amount effective to produce the desired benefits on the health and growth properties of poultry according to present invention. Preferably, an amount of from 10 to 400 µg, more preferably from 20 to 200 µg, most preferably from 50 to 100 µg carnitine, based on the amount of the free base, is injected per egg. Such injection may take place once or several times; preferably, one injection per egg is performed.

Suitable volumes that are injected are in the range of 0.01 to 1 ml. Preferably, a volume of 0.05 to 0.5 ml, most preferably a volume of 0.05 to 0.2 ml is injected.

The embryonated, incubating eggs according to the present invention can be injected any time during incubation prior to hatch. Incubation according to the present invention means storing the eggs under breeding conditions. The suitable breeding termperature range is well-known in the art; usually a temperature of or around 37°C is preferred. According to the present invention, eggs can be incubated at breeding temperature immediately post-laying. It is also possible according to the present invention, and is well-known in the art, to store eggs at appropriate humidity and appropriate low temperature immediately post-laying before starting the incubation. Eggs stored in this way can be preserved for at least two weeks with no reduction in hatchability prior to starting the incubation. Preferably, the eggs are injected at from 10 days of incubation up to 21 days. More preferably, the eggs are injected in between 15 to 20 days of incubation.

Machinery for in-ovo-injection according to the present invention is commercially available and is well-known from inoculation of eggs with vaccines. One acceptable egg injection apparatus is the INOVOJECT™ egg injection system manufactured and sold by Embrex Inc., Conneticut/U.S.A. The injection system can inoculate and transfer up to 50,000 eggs per hour. Needles are individually sanitized after each injection. Thus injection can be performed with high throughput at minimal cost per unit.

Preferably, other active ingredients such as, *e.g*., vaccines, therapeutics or vitamins are injected in admixture with the carnitine in a single all-in-one injection step. Since such injection application of therapeutic compounds is already routinely taking place in the poultry breeding business, the application of carnitine via in-ovo injection is not generating extra cost for processing the eggs for injection.

In a further possible, preferred embodiment of the present invention, the in-ovo-injection of carnitine in embryonated eggs as described in the foregoing is expediently combined with conventional feeding of a carnitine-supplemented diet after hatching. Thus maximum benefit is provided to the animals.

Another object of the present invention is the use of carnitine for the preparation of a liquid for delivering an effective amount of carnitine to an egg of poultry, characterized in that the egg is injected with said liquid.

The invention is further described in the subsequent examples.

### Examples

### Example 1 In-ovo Injection of Eggs

Approximately 4000 embryonated, freshly laid eggs of Cobb-500 strain of broiler chicken (obtained from Cobb Vantress of Siloam Springs, Arkansas) were pre-selected as to uniformity of weight and originated from breeder hens at approximately 35-45 weeks of the production cycle. The eggs were kept in a climatised incubation chamber at 37°C up to day 18 post-laying. Eggs were then injected with either 100 µl sterile physiological saline or 150 µg carnitine comprised in 100 µl sterile physiological saline on day 18 of incubation. Incubation was then carried on until hatch. Carnitine was commercially available L-Carnitine (trade name: Carnifeed, Lonza Inc., 100 % L-Carnitine). The eggs were injected with the automatic inovoject-system of Embrex, Inc. (maximum capacity: 50,000 eggs per hour). The system punches a tiny hole into the egg shell and then lowers a needle through the hole which delivers the therapeutic product to the embryo. Needles are automatically sanitized after each injection and provide for 100% inoculation.

### Example 2 Effect of Day 18 Carnitine Injection on Subsequent Broiler Performance

To assess the effect of the carnitine injection carried out according to Example 1, birds were selected at random from the birds originating from the Example 1 and raised up to 70 days. The birds were either placed in a commercial production environment (12 floor pens/trt; 50 birds/pen) or in metabolic chambers with normal feed but subnormal, controlled oxygen supply. The latter is an accepted testing model for trigger the ascites condition in predispositioned birds. Approximately 2400 chicks were allocated to floor pen groups as described below. Three hundred birds were selected at random and incubated in the metabolic chambers. Standard feeds were applied (similar to diets fed to commercial broiler chickens); and nutrient specifications are shown below. Feeds were mixed according to accepted mixing procedures such that treatment diets only differed in presence or absence of carnitine.

Carnitine injection proved to be beneficial in reducing mortality and improving body weight and feed conversion. Data were analyzed using the General Linear Model procedure of SAS (SAS, 1985). When a significant F statistic was noted by ANOVA, treatment means were compared using least square analysis variance.

Nutrient specifications of diets fed (approx. values) are provided in Table 1.

**Table 1**

| *Nutrient* | Starter (0-21d) | Grower (21-49 d) | Finisher (49-70 d) |
|---|---|---|---|
| Protein (%) | 22.67 | 20.2 | 17.33 |
| ME (kcal/kg) | 3075 | 3150 | 3225 |
| Lysine (%) | 1.240 | 1.105 | 0.955 |
| Methionine (%) | 0.525 | 0.480 | 0.340 |
| Methionine + Cystine (%) | 0.895 | 0.833 | 0.735 |
| Tryptophan (%) | 0.2 | 0.18 | 0.16 |
| Threonine (%) | 0.8 | 0.74 | 0.68 |
| Isoleucine (%) | 0.8 | 0.73 | 0.62 |
| Histidine (%) | 0.35 | 0.32 | 0.27 |
| Valine (%) | 0.9 | 0.82 | 0.7 |
| Leucine (%) | 1.2 | 1 | 0.85 |
| Arginine (%) | 1.25 | 1.1 | 1 |
| Phenylalanine (%) | 0.72 | 0.65 | 0.56 |
| Calcium (%) | 1 | 0.9 | 0.8 |
| Sodium (%) | 0.2 | 0.15 | 0.12 |
| Phosphorus (Avail %) | 0.45 | 0.35 | 0.3 |
| Fat, Crude (%, min) | 2 | 2 | 2 |
| Fiber, Crude (%, max) | 4 | 4 | 4 |

### 2.1 Floor pen studies:

Results are summarized below. Data are only given where probability levels were p≤0.10. In general, carnitine effects, though not always being statistically significant at p <0.05, were consistent across trials.

### 2.1.1 Body weight:

Main effect means of body weight (BW, in g) and weight gain (WG, in g) are given in Table 2. Number of days post-hatch are indicated, e.g., BW21: Hatched chicks were grown for 21 days prior to measurement.

**Table 2**

| Treatment | BW 21 | BW 49 | BW 70 | WG 0-21 | WG 21-49 | WG 49-70 |
|---|---|---|---|---|---|---|
| injected | 719.9 | 2940.7 | 4168.1 | 681.7 | 2220.8 | 1227.4 |
| not injected | 705.6 | 2897.4 | 4165.5 | 668.6 | 2191.8 | 1268.2 |

| | | | | | | |
|---|---|---|---|---|---|---|
| not-injected = control group only injected with sterile saline w/o carnitine | | | | | | |

At the end of the 70 days period post-hatch, the weight per 100 chicks started was 32.54 kg in case of the injected eggs, and 32.42 kg in case of the not-injected control group. Thus a single injection of a precise, small amount of carnitine during incubtion of the egg has far-reaching effect on further development of the hatched animal.

### 2.1.2 Mortality:

Mortality (M) is given as main effect mean in %. Number of days post-hatch are indicated, e.g., M21-49: percentage of death birds in the period of 21 to 49 days post-hatch.

**Table 3**

| Treatment | M 21-49 | M 49-70 | M 0-70 |
|---|---|---|---|
| injected | 4.66 | 14.33 | 21.92 |
| not injected | 4.75 | 14.50 | 22.17 |

### 2.1.3 3 Feed conversion ratio:

Main effect means for feed conversion ratio (FCR, in grams of feed per gram of gain) is given. Number of days post-hatch are indicated, e.g., FCR 0-49: FCR in the period 0 to 49 days post-hatch that chicks were grown. For further control, the effect of in-ovo injection was compared to that of feeding the same diet, except that this diet was supplemented with carnitine at 200 ppm. Feed conversion was improved by carnitine treatment, either fed or injected. The results from both methods of carnitine administration were consistent.

**Table 4**

| Treatment | FCR 0-49 | FCR 49-70 | FCR 0-70 |
|---|---|---|---|
| injected | 1.839 | 3.648 | 2.369 |
| not injected | 1.842 | 3.538 | 2.357 |
| fed | 1.817 | 3.535 | 2.331 |
| not fed | 1.863 | 3.652 | 2.396 |

### 2.2 Metabolic chamber studies:

Chicks were reared in air-tight chambers at subnormal oxygen supply simulating high altitude conditions. Feed and drinking water were provided for ad-libitum consumption. Chamber temperature was maintained at 30°C the first 5 days of age and at 27.8°C thereafter. Chambers were checked twice daily for bird mortality. Compressed dried air (7% relative humidity (rH), 17 % oxygen) was delivered to chicks in the chamber via polyethylene tubes. It was necessary to provide low rH-air to the chick chamber in order to hold the chamber at rH below 75%. The concentration of oxygen and carbon dioxide in air exiting each bird's chamber was monitored every 15-25 minutes using Ametek oxygen (accuracy ± 0.2%) and carbon dioxide (accuracy ± 0.3%) analyzers, respectively.

Main effect means for the various parameters are given.

Apart from body weight and body temperature at hatching (day 0; BW0, BT0), ascites score (AS) and hematocrit (packed cell volume; HCT) of chicks were determined at day 14. Blood (2 ml) was taken from the birds via venipuncture after which they were euthanized using CO₂ and necropsies were performed.

Hearts were weighed whole and then dissected for determination of ascites heart ratio. Heart sections were taken and fixed in 10% buffered neutral formalin for histopathologic examinantion. The results of heart dissection and histopathological analysis were summarized in the ascites score.

**Table 5**

| | BW 0 (g) | BT 0 (°F) | AS¹ | HCT² |
|---|---|---|---|---|
| Injected | 38.82 | 101.73 | 0.277 | 30.04 |
| not injected | 37.31 | 100.99 | 0.324 | 30.22 |
| fed | - | - | 0.303 | 30.25 |
| not fed | - | - | 0.297 | 30.00 |

| | | | | |
|---|---|---|---|---|
| ¹On a scale from 0-3 indicating no, slight, moderate, or severe ascites lesions, respectivelly. ²Percent packed cells. | | | | |

Injection of carnitine decreased hematocrit and the ascites score significantly, consistent with a further non-injected control group to which L-carnitine at 200 ppm (coated on silica particles) was applied via the feed.

## Claims

1. Use of carnitine or an alkanoyl-carnitine or a salt thereof for producing an injection solution for in-ovo injection of embryonated eggs of poultry in between 10 to 21 days of incubation for preventing or alleviating ascites in poultry .

2. Use according to claim 1, **characterized in that** the embryonated eggs were laid by a breeder hen.

3. Use according to one of the preceding claims, **characterized in that** the carnitine injected is L-carnitine or a salt thereof.

4. Use according to one of the preceding claims, **characterized in that** an amount of from 10 to 400 µg of carnitine or an alkanoyl-carnitine or a salt thereof is injected.

5. Use according to claim 4, **characterized in that** an amount of from 20 to 200 µg of carnitine or an alkanoyl-carnitine or a salt thereof is injected.

6. Use according to one of the preceding claims, **characterized in that** a volume of 0.01 to 1 ml is injected.

7. Use according to one of the preceding claims, **characterized in that** the egg is injected once with a single dose of carnitine or an alkanoyl-carnitine or a salt thereof.

8. Use according to one of the preceding claims, **characterized in that** the carnitine or alkanoyl-carnitine is in admixture with at least one other substance selected from the group comprising therapeutic substances, vaccines and vitamins.

## Patentansprüche

1. Verwendung von Carnitin oder einem Alkanoylcarnitin oder einem Salz davon zur Herstellung einer Injektionslösung für die in-ovo-Injektion von embryonierten Geflügeleiern mit einer Inkubationsdauer zwischen 10 und 21 Tagen zur Vorbeugung oder Linderung von Ascites in Geflügel.

2. Verwendung nach Anspruch 1, **dadurch gekennzeichnet, daß** die embryonierten Eier von einer Bruthenne gelegt wurden.

3. Verwendung nach einem der vorhergehenden Ansprüche, **dadurch gekennzeichnet, daß** es sich bei dem injizierten Carnitin um L-Carnitin oder ein Salz davon handelt.

4. Verwendung nach einem der vorhergehenden Ansprüche, **dadurch gekennzeichnet, daß** eine Menge von 10 bis 400 µg Carnitin oder eines Alkanoylcarnitins oder eines Salzes davon injiziert wird.

5. Verwendung nach Anspruch 4, **dadurch gekennzeichnet, daß** eine Menge von 20 bis 200 µg Carnitin oder eines Alkanoylcarnitins oder eines Salzes davon injiziert wird.

6. Verwendung nach einem der vorhergehenden Ansprüche, **dadurch gekennzeichnet, daß** ein Volumen von 0,01 bis 1 ml injiziert wird.

7. Verwendung nach einem der vorhergehenden Ansprüche, **dadurch gekennzeichnet, daß** dem Ei einmal eine Einzeldosis Carnitin oder eines Alkanoylcarnitins oder eines Salzes davon injiziert wird.

8. Verwendung nach einem der vorhergehenden Ansprüche, **dadurch gekennzeichnet, daß** das Carnitin oder Alkanoylcarnitin in Abmischung mit mindestens einer weiteren Substanz aus der Gruppe Therapeutika, Impfstoffe und Vitamine vorliegt.

## Revendications

1. Utilisation de carnitine ou d'une alcanoyl-carnitine ou d'un sel de celles-ci pour la production d'une solution injectable pour l'injection in ovo d'oeufs embryonnés de volailles entre les jours 10 à 21 de l'incubation pour la prévention ou l'atténuation de l'ascite chez la volaille.

2. Utilisation selon la revendication 1, **caractérisée en ce que** les oeufs embryonnés ont été pondus par une poule d'élevage.

3. Utilisation selon l'une des revendications précédentes, **caractérisée en ce que** la carnitine injectée est la L-carnitine ou un sel de celle-ci.

4. Utilisation selon l'une des revendications précédentes, **caractérisée en ce qu'**on injecte une quantité allant de 10 à 400 µg de carnitine ou d'une alcanoyl-carnitine ou d'un sel de celles-ci.

5. Utilisation selon la revendication 4, **caractérisée en ce qu'**on injecte une quantité allant de 20 à 200 µg de carnitine ou d'une alcanoyl-carnitine ou d'un sel de celles-ci.

6. Utilisation selon l'une des revendications précédentes, **caractérisée en ce qu'**on injecte un volume allant de 0,01 à 1 ml.

7. Utilisation selon l'une des revendications précédentes, **caractérisée en ce qu'**on injecte l'oeuf une seule fois par une dose unique de carnitine ou d'une alcanoyl-carnitine ou d'un sel de celles-ci.

8. Utilisation selon l'une des revendications précédentes, **caractérisée en ce que** la carnitine ou l'alcanoyl-carnitine est en co-mélange avec au moins une autre substance choisie parmi le groupe constitué par les substances thérapeutiques, les vaccins et les vitamines.
